# EUROPEAN PATENT SPECIFICATION

(11) **EP 2 967 744 B1**
(45) Date of publication and mention of the grant of the patent: **18.10.2017**
(21) Application number: 14763334.1
(22) Date of filing: 10.03.2014
(51) Int. Cl.: A61B 18/18, A61N 5/06, A61B 18/00, A61B 18/14, A61B 18/20, A61N 7/00

(54) **SKIN TREATMENT APPARATUS**
HAUTBEHANDLUNGSVORRICHTUNG
APPAREIL DE TRAITEMENT DE LA PEAU

(30) Priority: 14.03.2013 US 201361781166 P
(43) Date of publication of application: 20.01.2016
(73) Proprietor: Syneron Medical Ltd., 20692 Yoqneam Illit (IL)
(72) Inventor: ROSENBERG, Avner, 36578 Bet Shearim (IL); ADANNY, Yossef Ori, 37367 Mitzpe Ilan (IL); ISRAELI, Roy, 3455540 Haifa (IL)
(74) Representative: ZBM Patents - Zea, Barlocci & Markvardsen
(86) International application number: PCT/IL2014/000016
(87) International publication number: WO 2014/141229

(56) References cited:
- WO-A1-95/12357
- WO-A1-2008/124839
- US-A- 6 165 170
- US-A1- 2007 185 553
- US-A1- 2010 063 487
- US-A1- 2010 063 487
- US-A1- 2011 172 746
- US-A1- 2011 172 746
- US-A1- 2012 071 794

## Description

### TECHNOLOGY FIELD

The apparatus relates to the field of cosmetic skin treatment apparatuses and in particular to hair removal apparatuses.

### BACKGROUND

Cosmetic skin treatment apparatuses and in particular apparatuses for hair removal with the help of light are known for some time. These apparatuses use coherent (laser) or non-coherent (IPL) light to deliver high intensity light pulse to the skin. The light heats the skin and growing on it hair. The hair that usually is darker than the skin is absorbs a larger amount of heat and is heated to temperatures higher than the skin is heated. The temperature of the hair could reach 50 or 60 degrees Celsius and is sufficient to destroy the hair follicle, which practically removes the hair. The skin, however, is also heated to a relatively high temperature and to avoid skin burns, the surface of skin is cooled by different methods that include application of liquid gases, thermoelectric cooling devices and water or air cooling. Such a device comprising a reciprocating light source, is disclosed in WO 2008/124839.

The hair removal apparatus usually includes an applicator designed to be held by the caregiver and applied to the treated segment of skin. The applicator has a transparent window through which the light provided by a source of light is applied to the skin. A typical size of the light application area of the applicator is between 10x20mm to 30x30mm. The caregiver attaches the application area firmly to the skin, activates the light source and applies one or more pulses of light to the treated segment of skin. Following this the caregiver repositions the applicator to the next segment of skin to be treated and repeats the process. Alternatively, the caregiver can move the applicator with the application area moving continuously over the skin surface while the light source is periodically activated to expose a segment of skin.

Some apparatuses apply to the skin a combination of light and RF (radio frequency) energy. Combination of light and RF energy facilitates faster heating of deeper skin layers where the hair follicle resides and improves the hair removal process. Application of RF also allows reducing the skin surface temperature, although the treatment process is similar to the treatment by applicators providing light only. The caregiver attaches the application area firmly to the skin, activates the light and RF source and applies one or more pulses of combined energy to the treated segment of skin. Following this the caregiver repositions the applicator to the next segment of skin to be treated and repeats the process. Alternatively, the caregiver can move the applicator with the application area moving continuously over the skin surface while the light and RF sources are periodically activated.

### SUMMARY

Presented is an apparatus for treatment of large skin areas that includes a treatment head or applicator that could be displaced in one or two directions. The treatment head is configured to apply to skin different treatment energies. The treatment head could be displaced or moved manually or automatically on a frame configured to be applied to a large skin segment. The frame could include guides on which the treatment head could reciprocate. The frame includes a pair of handles located on opposite sides of the frame. The handles facilitate the frame handling including positioning on a surface of skin to be treated.

The treatment head includes one or more sources of treatment energy. Such energy could be light energy, RF energy, ultrasound energy and a combination of the above energies. The treatment energies are applied to the treated skin segment in course of the treatment head reciprocating movement.

A controller governs operation of the apparatus. The movement of the applicator and in particular automated and controlled movement along the skin open possibilities for optimized skin treatment and inclusion of various sensors. A number of various sensors, such as temperature sensors, impedance detecting circuits, encoders, and cameras communicated with the controller and provide feedback on the skin treatment process. Some of the sensors could be configured to measure skin properties before the treatment and some sensors could be configured to measure skin properties after the treatment. Based on this feedback, the controller could change the skin treatment parameters. The skin treatment parameters could be changed in course of the treatment in a dynamic mode or between the treatments.

A cooling device is associated with the treatment head and configured to cool the treated skin segments.

The invention is defined in the claims, other embodiments being merely exemplary.

### DETAILED DESCRIPTION

The apparatuses and applicators described are built to treat segments of skin with an area matching the size of the transparent window through which the light provided by a source of light is applied to the skin. Removal of hair from a relatively large skin area, for example chest, back or leg becomes a tedious task. The caregiver has to move manually an applicator with relatively small application area, for example, 10x10sqmm over a large skin area to be treated. In addition to hard work it is difficult for the caregiver to deliver a uniform coverage of the skin. There is a risk of delivering too much energy to some skin segments and not delivering enough energy to other skin segments. The result might be insufficient treatment in some skin segments and adverse effects in other skin segments.

The existing skin treatment applicators use light or other energy delivery surface also as a skin cooling surface. Use of the same surface for a number of actions does not support optimal control of the cooling; it depends on the caregiver attachment time of the applicator on the skin before delivering the light or other energy pulse. US Patent No. 6,383,176 to Connors disclose a manually displaced skin treatment device where the light energy delivery surface and skin cooling surface are different and separate surfaces. Since the device is manually displaced it does not support optimal control of energy delivery and cooling times.

Control of the skin treatment process by such applicators is complicated. Inclusion of various sensors to control for example, the skin temperature in course of the treatment process is difficult in such applicators. Treated skin properties are almost impossible to determine, although it would be beneficial to know skin properties before, during and after application of the treatment energy.

These and other problems could be resolved by optimal timing of delivery of the treatment RF energy, ultrasound energy, cooling and light energy to obtain optimal temperature distribution inside the tissue and optimal skin treatment process. It is difficult to implement all of these functions in an applicator whose movement on the skin is done manually by the caregiver. For example, it could be optimal to cool the skin first for half a second before the application of the energy. This could also be the time between two successive light or RF pulses. A caregiver cannot perform this kind of treatment and maintain optimal timing just by moving the applicator and pushing the trigger.

The present apparatus offers an efficient way for conducting a skin treatment process. It facilitates inclusion of different sensors into the apparatus, optimization of the skin treatment process and in particular treatment of large skin areas.

### BRIEF DESCRIPTION OF THE DRAWINGS

FIG. 1 is a simplified side view illustration of the apparatus for treatment of a large skin area according to an example;
FIG. 2 is a simplified top view illustration of the apparatus for treatment of a large skin area according to an example;
FIG. 3 is a simplified illustration of a cross section of the treatment head of the apparatus for treatment of a large skin area according to an example;
FIG. 4 is a simplified illustration of a treatment head with cooling devices of the apparatus for treatment of a large skin area according to an example;
FIG. 5 is a simplified illustration of a treatment head with a pair of RF electrodes of the apparatus for treatment of a large skin area according to an example;
FIG. 6 is a simplified illustration of a treatment head with imaging devices of the apparatus for treatment of a large skin area according to an example;
FIG. 7 is a simplified illustration of the apparatus for treatment of a large skin area including a controller according to an example;
FIG. 8 is a simplified illustration of the treatment head with RF electrodes of the apparatus for treatment of a large skin area according to an example;
FIG. 9 is a simplified illustration of the treatment head including RF electrodes of the apparatus for treatment of a large skin area according to an example;
FIG. 10 is a simplified illustration of the treatment head including different sensors and RF electrodes of the apparatus for treatment of a large skin area according to an example; and
FIG. 11 is a simplified top view illustration of the apparatus for treatment of a large skin area according to an example.

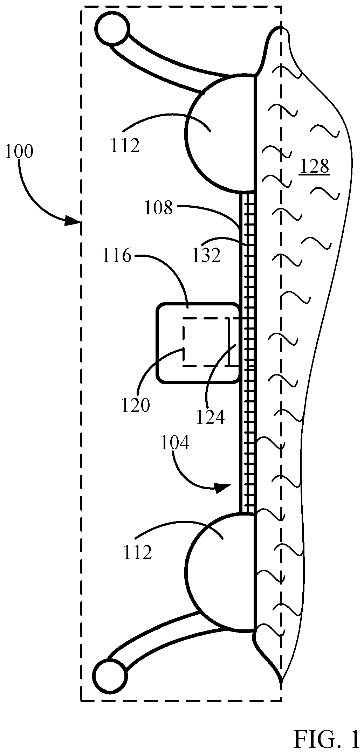

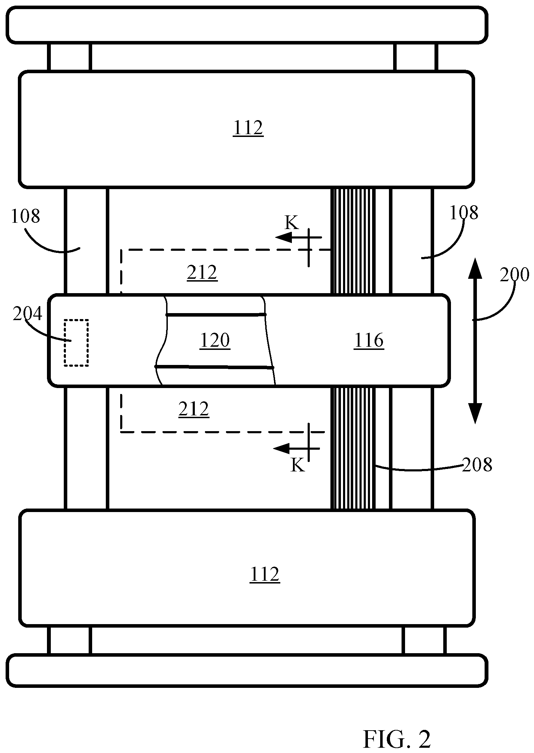

Reference is made to FIG. 1, which is a simplified side view illustration of the apparatus for treatment of large skin area according to an example. Apparatus 100 includes a frame 104 configured to be applied to a large skin segment. Frame 104 could include one or more guides 108 and at least one handle 112, or a pair of handles located on opposite sides of frame 104. Frame 104 could be made sufficiently rigid or equipped with vacuum cups facilitating stable fixation of frame on skin in course of the treatment process. A treatment head or applicator 116 is configured to reciprocate along guides 108. Treatment head 116 could include one or more light emitters 120 and a light guide 124 configured to deliver light energy to a treated skin segment 128. Generally, the light guide 124 could be made of glass or quartz, also in some examples, as it will be shown below the light guide 124 could be made from sapphire. The treatment head 116 could include a mechanism configured to provide the treatment head position along guides 108. Such mechanism could be for example, an encoder. In one example, the mechanism could be a rotary encoder 204 (FIG. 2) associated or connected to the treatment head 116. Alternatively, it could be a linear encoder or a simple scale 132 attached to guides 108 with a readout photo-element located on the treatment head 116.

Treatment head 116 could be configured to maintain in course of the treatment head 116 reciprocating movement a permanent or intermittent contact with the treated skin segment. Contact with the treated skin segment could facilitate delivery of treatment energy to the treated skin segment, skin temperature measurement and other skin treatment processes. A spring or solenoid (not shown) could be used as a mechanism configured to facilitate and maintain a permanent or intermittent contact of the treatment head 116 with the treated skin segment.

The reciprocating movement of treatment head 116 shown in FIG. 2 by arrow 200 could be performed manually or with the help of a mechanism configured to provide to the treatment head 116 reciprocating movement. Light energy sources 120 could be configured to operate in course of the treatment head 116 reciprocating movement and deliver the light energy to the treated skin segment. Rotary encoder 204 (FIG. 2) or linear encoder 132 facilitate manual movement by providing the treatment head 116 location and avoiding double exposure by light energy pulses of the same skin area to be treated or underexposure of the skin area. The mechanism configured to provide to the treatment head reciprocating movement could include electrical or pneumatic motor located for example, in one or both handles 112 of frame 104 and a belt 208 or other type of transmission conveying the movement to treatment head 116.

The treatment head or applicator 116 could also include a skin cooling device 212 configured to cool the treated skin segment. The cooling device 212 could be attached to the treatment head 116 and move with the treatment head in a reciprocating type movement as shown by arrow 200. Cooling device 212 could be attached on one or both sides of treatment head 116. When cooling device 212 is attached to the leading edge of treatment head 116 it will first cool a skin segment to which light energy will be applied. When cooling device is attached to the trailing edge of treatment head 116 it will cool a skin segment to which light energy has been already applied. A combination of a cooling device attached to both leading and trailing edges of treatment head 116 could also be implemented. In one example, light guide 124 could be cooled. Such applicator structure could be configured to support the treated skin segment cooling before application to it of light energy, concurrently with the application of light energy and after the application of light energy to the treated skin segment. For example, light guide 124 could be made from sapphire, which is known to have good heat conducting properties Cooling of the treated skin segment by any of the cooling devices 212 configuration (leading edge, trailing edge or light guide) can be done for example, by contact of a solid element with the skin. The solid element could be made of ceramics, glass, and heat conducting plastic or metal. The solid cooled element can be cooled by flow of a fluid, which can be air, water, oil or other fluids, or it can be cooled by thermo-electric-cooler (TEC). According to another example, non-contact cooling could be used to cool the treated skin segment. The non-contact cooling could be achieved by directing a spray of cold liquid or gas to the treated skin segment.

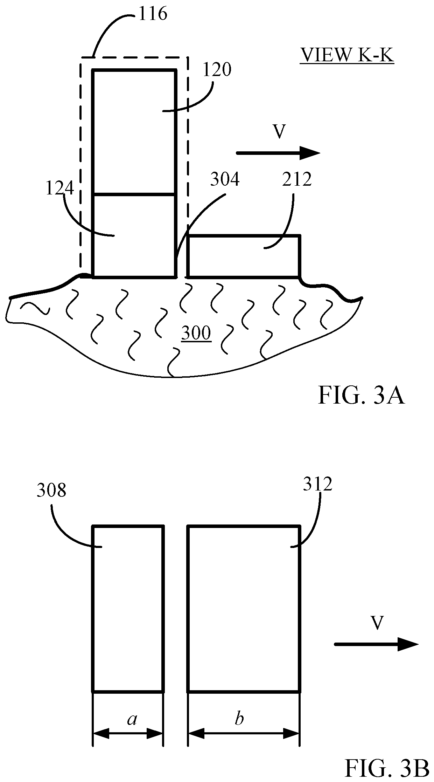

The size of the cooling device, light pulse rate and movement velocity are optimally matched to get the desired temperature profiles in the treated skin. Such matching could be achieved by automatic and controlled movement of the treatment head or applicator 116. FIG. 3 is a simplified illustration of a cross section of the treatment head of the apparatus for treatment of a large skin area according to an example. Light energy source 120 delivers light energy that could be in visible or infrared region of the spectrum, through a light guide 124 to skin or a skin segment 300 to be treated. Light guide 124 could be in contact with the skin segment to be treated or there could be a gap between the light guide and the skin segment to be treated. A cooling device 212 is located adjacent the leading edge 304 of treatment head 116 that moves in the direction indicated by arrow V. Cooling device 212 is illustrated as a plate being in contact with skin 300. When cooling device 212 is in contact with the treated skin segment, device 212 could be cooled by a cooling fluid like water, oil or other fluids or by a thermo-electric cooler (TEC). When cooling device 212 has no contact with the treated skin segment, device 212 could be configured to include emitters of liquid or liquid gas which would cool the treated skin segment. For simplicity of the explanation the mechanical structure of the treatment head fixing all elements together is not shown. Light energy source 120 could be a non-coherent intense pulse light (IPL) source such as a Xenon lamp, an LED, a laser diode or a laser.

The light energy used for skin treatment is absorbed exponentially when propagating into the skin, typically delivering more energy to the upper skin layer. However, in most cases, and specifically for hair removal, the heating is required in deeper skin (1-3mm deep) layers. The heating of upper skin is a major cause of discomfort, pain, and sometimes adverse events of burns. Excessive heating of the upper skin layer limits the possibility of delivering enough energy to deep skin and to get good treatment effects, and in particular hair removal.

FIG. 3B illustrates an example of the "footprint" 308 of light energy source 120 and 312 of skin cooling device 212 on skin 300. The velocity with which treatment head 116 reciprocates is **V,** the light energy source width is a, and the skin cooling section width is b. Cooling device 212 is attached to the leading edge of applicator 116. The time of cooling of a certain treated skin segment before it is exposed to light energy is **b/V.** A typical numerical example of a practical implementation of a treatment head: **a**=5mm, **b**=10mm, **V**=15mm/sec. Accordingly, the cooling time before the light pulse is 1sec. To completely expose the skin segment to be treated the light energy source has to apply to this skin segment at least three light energy pulses per second. Control of the skin heating process by proper dimensioning of the light guide 124 and cooling section 212 facilitates reduction or even eliminates discomfort, pain, and some adverse treatment effects. Separation between the light guide, coupling light energy to the skin and the cooling section further support proper timing and maintenance of light energy and cooling to skin application.

As disclosed above, a combination of a cooling device attached to both leading and trailing edges of treatment head 116 could also be implemented. Operation of the leading and trailing edge cooling devices 212 could be further enhanced by cooling of light guide 124 to serve as a cooling device. Different combinations of the cooling devices operation modes could be implemented and the cooling time could be distributed between the cooling devices. For example, if the leading edge cooling device and a cooled light guide are operative to cool the treated skin segment, and assuming the light pulse is very short compared to **a/V**, there is a difference in cooling time at the leading and trailing edge of skin section 308. To reduce this difference, according to an example, a can be selected to be as small as possible. This is also advantageous for reducing pain. Lower practical limit of a is determined by the light scattering losses inside the skin. Below **a**=2mm, light scattering loss could be significant, so value of a between 2mm to 8mm, or between 4mm to 6mm seems optimal. Different cooling time proportions between different combinations and operation modes of the cooling devices are possible.

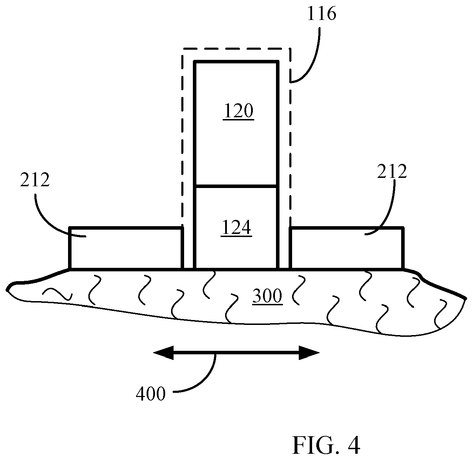

In one example, illustrated in Fig. 4, cooling devices 212 are attached on both sides of the treatment head i.e., to both leading and trailing edges of treatment head 116. The two cooling devices 212 could be configured to cool the treated skin segment before and after the application of light energy pulses. Cooling after application of the light energy pulses could diminish some residual pain sensation that could be occasionally felt after the treatment. In one example cooling devices 212 are identical. Making the cooling devices as illustrated in in Fig. 4 identical facilitates skin treatment by a reciprocating movement of treatment head 116 indicated by arrow 400. The treated skin segments get the same amount of light energy and identical cooling devices performance regardless the direction the treatment head is displaced or moves in course of the treatment.

Typical parameters of a treatment head with cooling devices would be in the range of: **a -** 2mm to 20mm or more typically between 3mm to 10mm or 4mm to 6mm; **b -** 3mm to 30mm or more typically 5mm to 15mm; **V -** 5mm/sec to 50mm/sec; and cooling sections temperature 5 to 25 degrees Celsius.

In one example, light guide 124 could also be cooled and be in contact with the treated segment of skin to provide continuous cooling of the treated skin segment until the treatment light energy pulse arrives. When the light guide is cooled, there could be a difference in the ratio of the light energy footprint that becomes equal to the cooling footprint, since the skin under the leading edge of the light guide 124 gets less cooling than the skin under the trailing edge of the light guide 124. The difference in the cooling times is **a/V.** By making a smaller than b we make this difference smaller. This difference could be reduced by implementing the dimension a substantially smaller than the dimension **b** (**a**<< **b**). In the example, where there could be a gap between the light guide 124 and the skin segment to be treated a non-contact cooling of the treated skin segment could be implemented.

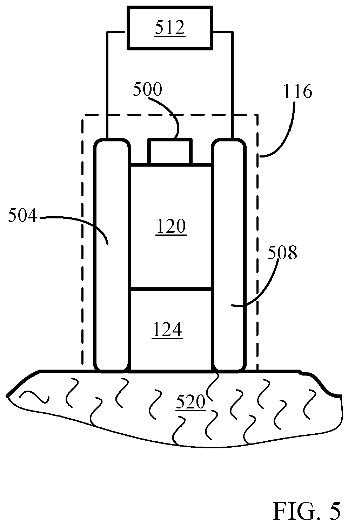

One or more different sensors could be located in treatment head 116. Some of the sensors, for example 500 (Fig. 5) could be configured to measure the treated skin segment 520 temperature. Such sensor could be an infrared (non-contact) temperature sensor, a thermistor, or a thermocouple. Alternatively, a pair of electrodes 504 and 508 could be applied to the skin and fed with a low voltage (for example, 1 to 5 volt) RF signal. A simple electronic circuit 512 could be configured to operate at a number of RF frequencies and extract from the treated skin segment impedance variations of the treated skin segment, which can be related to temperature. Electronic circuit 512 could be configured to operate in course of treatment head 116 movement or in a static treatment head 116 condition. The sensors could be mounted inside treatment head 116 or on one or both sides of the light energy guide 124 and operate in course of the treatment head 116 movement. In one example, the sensors could be ultrasound sensors measuring ultrasound propagation speed in the treated skin segment and extracting from the treated skin segment ultrasound propagation speed variations of the treated skin segment temperature.

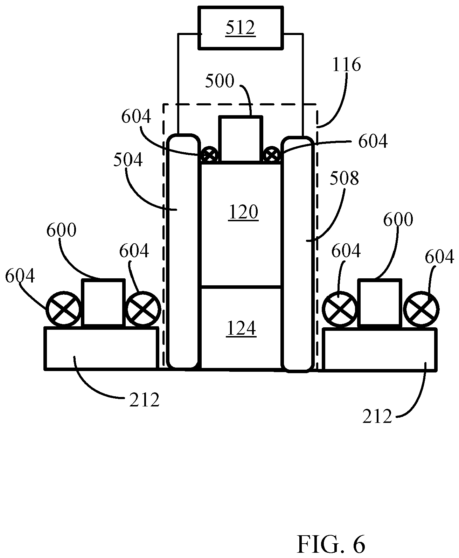

Some of the sensors could be one or more optical sensors. Such sensors could sense the treated skin segment properties. Such sensors also could be optical sensors 600 (FIG. 6) configured to form an image of the treated skin segment and assess the treated skin segment properties. For example, one optical sensor 600 could be located in one side (e.g. leading edge) of the light energy guide and another optical sensor 600 could be mounted at the other side (e.g. trailing edge) of the light energy guide. Both sensors could be configured to explore skin properties including forming an image of the treated skin segment before and after the application of the light energy. Both devices could operate in course of treatment head or applicator 116 movement along the treated segment of the skin and both devices could provide images of the treated skin segment.

Light sources 604 could be configured to illuminate the skin segments used to facilitate assessment of the treated skin segment properties. Light sources 604 could be configured to illuminate the treated skin segment to facilitate formation of an image of the treated skin segment. According to one example, light sources 604 could be a white light "Light Emitting Diode" (LED) such as for example, Luxeon series White LEDs commercially available from Philips Lumileds Lighting Company, San Jose, California USA or similar. In one example, light source 604 could be one or more LEDs emitting light of different colors. The colors could be selected arbitrarily and be Red, Green, Blue (RGB), Orange, Yellow and others. In one example, light sources 604 could be a combination of white and color LEDs. Light sources 604 could be configured to operate in pulse or continuous mode, as it could be best adapted to explore particular skin properties.

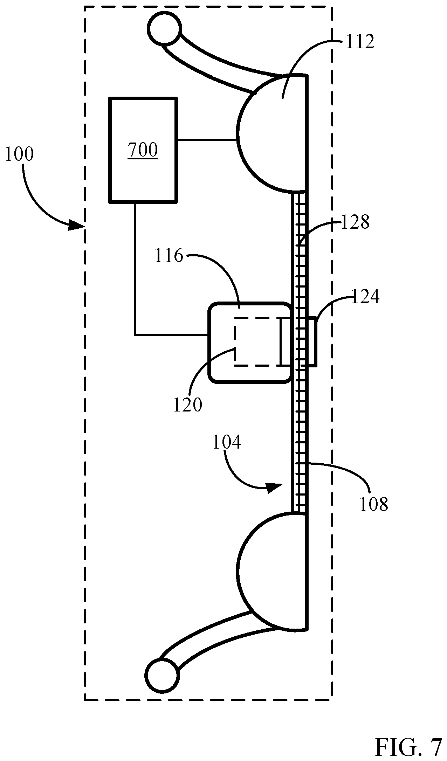

Apparatus 100 could also include a controller 700 (FIG. 7). Controller 700 could be configured to communicate with at least one of the sensors 500 and 600 and electronic circuit 512 and based on information received from one of the sensors change the treatment head 116 movement speed V, change the value or other parameters of the light energy or the RF energy coupled to the skin, ultrasound energy coupled to the skin, operate the skin cooling device 212 and control different drivers or sources of energy such as RF electrodes electronic driving circuit or a generator of RF energy, light source power supply and other function or devices that could be needed or engaged in the process of skin treatment. Skin treatment and in particular cosmetic skin treatment are usually performed in accordance with a skin treatment protocol. One or more of such skin treatment protocols could be prepared ahead of time and loaded into the memory of controller 700. Accordingly, controller 700 could also be configured to store these treatment protocols and provide to the light source and the RF electrodes energy in accordance with the treatment protocol. Controller 700 could also be configured to account for the input from different sensors and vary/change in course of treatment according to inputs from sensors some of the treatment protocol parameters.

It has been theoretically and experimentally discovered that delivery of RF energy to the skin during relatively long time supports the optimal temperature distribution inside the skin. In some cases delivery of RF energy could be combined with the cooling of upper skin layer. On the other hand, in some cases it is preferred to have a much shorter light energy pulse. The physical reason is the selective absorption of light. More light energy is absorbed in the hair follicles, raising them to higher temperature than the surrounding tissue to obtain the selective effect for hair removal. Heat conduction reduces the temperature difference therefore reduces the selective treatment of hair. Short light pulses are helpful in improving selective effect of hair removal. Existing skin treatment applicators do not possess geometry optimal for long RF energy application times and short light energy pulses. It is difficult if not impossible to implement accurate control of the time the applicator is applied to the skin, RF energy and light energy application timing. Existing manually displaced skin applicators do not support implementation of effective skin cooling from the moment of the applicator to skin application and through the end of the treatment. The automatic and controlled movement of the treatment head or applicator 116 facilitates accurate and optimal timing of the cooling, RF and light energies application.

Operation of the pair of RF electrodes 504 and 508 that, as explained above, could be fed by a low voltage to measure the treated skin segment impedance and determine the treated skin segment temperature, could be enhanced by supplying to them higher magnitude RF energy for example, voltage of about 50 to 500 volt. Such RF energy could be applied to the treated segment of skin in addition to light energy and also heat the treated skin segment. The RF energy at different RF frequencies could be applied to the treated skin segment in course of the treatment head movement and it could be applied before, concurrently or after application of light energy pulses. Different overlapping in time applications of RF energy and light energy are possible. The RF energy could be supplied in pulse mode or in continuous mode. The RF electrodes electronic driving circuit could be configured to measure skin impedance. Skin impedance measurement could be performed in one or more RF frequencies applied to the treated skin segment 704. Controller 700 could be configured to extract from the skin impedance the skin temperature variations during treatment head or applicator 116 movement.

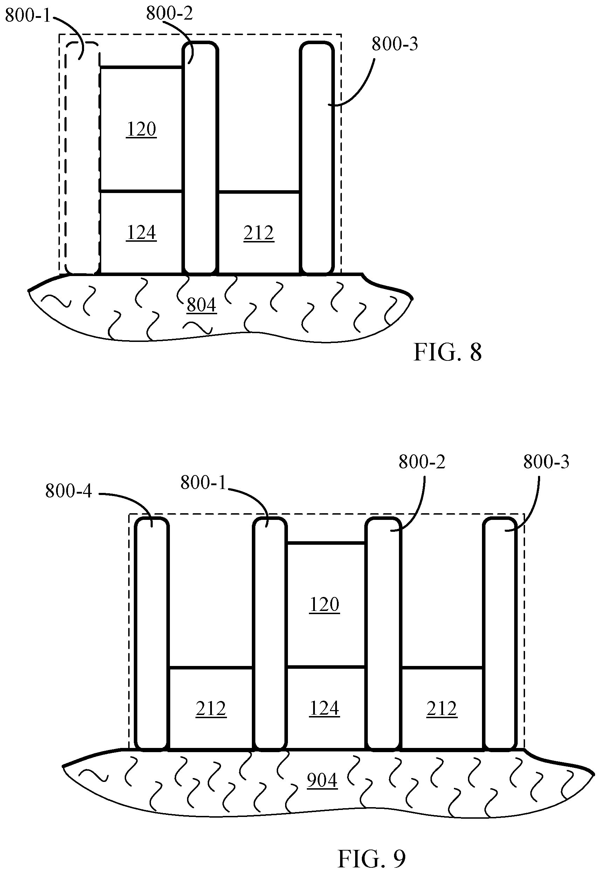

The pair of RF electrodes 800 similar to electrodes 504 and 508 could be located on both sides of a light guide, on both sides of a cooling device (FIG. 8), and a combination of the above (FIG. 9). The pair of RF electrodes 800-1 and 800-2 as well as additional electrodes 800-3 and 800-4 could be configured to maintain in course of the treatment head 116 movement a permanent or intermittent contact with the treated skin segment. When the electrodes are in contact with the treated skin segment they apply to it treatment RF energy. A spring or solenoid (not shown) could serve as a mechanism configured to maintain the treatment head with the skin contact and could be used to facilitate and maintain a permanent or intermittent contact of the electrodes 800 with the treated skin segment. RF energy could be supplied to any pair of RF electrodes 800, for example, to electrodes 800-1 and 800-2, or between pair of electrodes 800-3 and 800-4 or any other electrodes combination.

Electronic driving circuit could drive RF electrodes 504, 508, and 800 in various ways. These could be independent RF electronic driving circuit or one common electronic driving circuit that distributes RF energy between the electrodes. For example, the RF energy may be switched between electrodes 504 and 508, and/or 800-1 and 800-3. Other possible combination of RF energy supply to the electrodes could be used. According to an example, RF energy could be delivered continuously between electrodes 800-2 and 800-3 (FIG. 9) when treatment head or applicator 116 is moving to the right, and between 800-1 and 800-4 when treatment head or applicator 116 is moving to the left. RF energy could be applied ahead of the light energy, optionally together with cooling, so when the light guide or light energy output area arrives at a skin segment to which it delivers the light energy pulse, the skin segment could already be brought to the optimal temperature profile within the skin. According to an example, RF can be driven between electrodes 800-2 and 800-3 (FIG. 9), optionally in pulsed mode, optionally with at least partial overlap with the light energy pulse to facilitate obtaining effects specific to simultaneous application of light and RF energies. Optionally, additional heating and cooling can be provided to the skin after the light energy pulse by the pair of electrodes located behind with respect to the direction of movement. Typical RF energy parameters could be: frequency 100kHz to 50MHz, or 300kHz to 10MHz and RF power from 5W to 500W.

In some examples therapeutic ultrasound applied by one or more ultrasound transducers, could be used to heat the skin and in particular the hair follicle typically located 1 to 2 mm below the skin surface. This ultrasound skin segment heating could be combined, as disclosed above with light treatment and cooling of the upper skin layer.

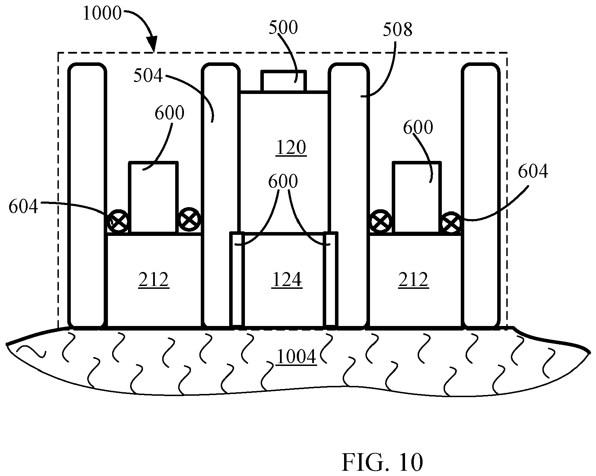

FIG. 10 is a simplified illustration of the treatment head including different sensors and RF electrodes of the apparatus for treatment of a large skin area according to an example. Treatment head 1000 could include one or more light emitters 120 and a light guide 124 configured to deliver light energy to a treated skin segment 1004. Light guide 124 could be in contact with the skin segment to be treated or there could be a gap between them. A pair of RF electrodes 504 and 508 or more than a pair of RF electrodes could be located at both the leading and trailing edges of light guide 124. In one example, RF electrodes could be located on both edges of the cooling devices 212. The RF electrodes could be configured to apply RF energy to the treated skin segment 1004 and maintain in course of the treatment head 1000 movement a permanent or intermittent contact with the treated skin segment. When the electrodes are in contact with the treated skin segment they apply to it treatment RF energy. A spring or solenoid (not shown) could be used to maintain the treatment head with the skin contact could be used to facilitate and maintain a permanent or intermittent contact of the electrodes 504 and 508 with the treated skin segment.

One or more different sensors could be located in treatment head 1000. Some of the sensors, for example 500 could be configured to measure the treated skin segment temperature. Such sensor could be an optical sensor, an infrared (non-contact) temperature sensor, a thermistor, or a thermocouple. The optical sensor could be configured to sense additional to the temperature skin properties, for example, skin color. Alternatively, a pair of electrodes 504 and 508 could communicate with controller 700 that among others could include a simple electronic circuit, similar to circuit 512 that could be configured to operate at a number of RF frequencies and determine the treated skin segment impedance and accordingly the treated skin segment temperature. The sensors could be mounted inside treatment head 1000 or on one or both sides of the light energy guide 124 and operate in course of the treatment head 1000 movement.

Some of the optical sensors could be one or more optical sensors configured to sense skin color. Some of the optical sensors could be configured to operate as imaging devices 600 configured to form an image of the treated skin segment and assess the treated skin segment properties. For example, some of the optical sensors configured to operate as imaging devices could be located and configured to image the treated skin segment located under light energy guide 124.. According to another example, one optical sensor 600 configured as an imaging device could be located in one side of the light energy guide 124 and another imaging device 600 could be mounted at the other side of the light energy guide 124. Both optical sensors 600 operating as imaging devices could be configured to explore skin properties, for example, skin color before and after the application of the light energy. Both devices could operate in course of treatment head or applicator 1000 movement along the treated segment of the skin 1004. According to one example, cooling devices 212 could be made from glass and optical sensors 500 or 600 configured as imaging devices could be mounted over such cooling devices 212 on both sides of the light energy guide 124. Both optical sensors 600 could be configured to explore skin properties, for example, skin color before and after the application of the light energy. Both optical sensors could operate in course of treatment head or applicator 1000 movement along the treated segment of the skin 1004.

Treatment head 1000 could include a mechanism configured to provide the treatment head position along guides 108. The mechanism could be similar to the described above rotary and linear encoders.

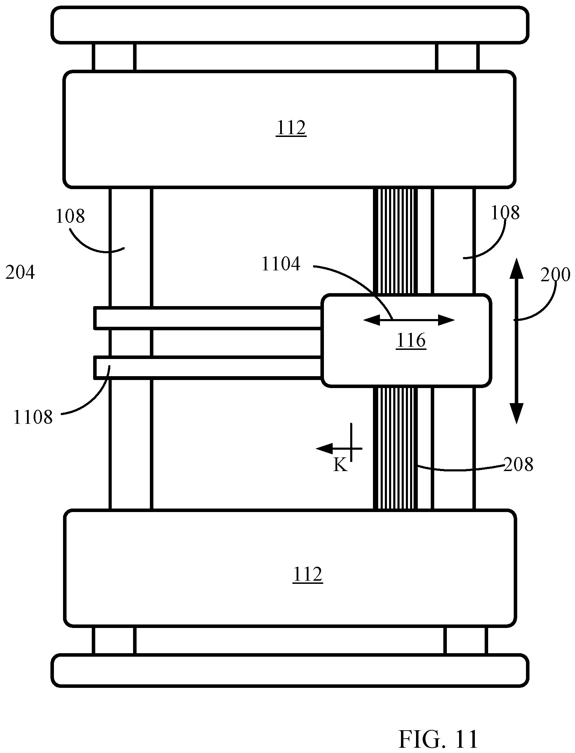

FIG. 11 is a simplified top view illustration of the apparatus for treatment of a large skin area according to an example. Apparatus 1100 is similar to apparatus 100 and in addition to it includes one or more guides 1108. Treatment head 116, or any one of treatment heads described could be configured to move in both direction indicated by arrow 200 and in a direction indicated by arrows 1104. Automated movement of treatment head 116 or similar in both directions supports accurate skin treatment protocol with application of proper values of treatment energies and skin cooling. All other elements described in different configurations are mutatis mutandis applicable to apparatus 1100.

The present apparatus is an apparatus for cosmetic skin treatment of large skin segments. In some examples, for treatment of even larger skin segments than skin segments determined by the size of the apparatus frame upon completion of a treated skin segment treatment, the apparatus could be repositioned to treat a next large skin segment. Apparatus repositioning could be made fairly accurate, since usually signs of the previous treatment could be present on the skin and the apparatus or the frame could be repositioned to avoid overlapping the already treated skin segment.

It will be appreciated by persons skilled in the art the present apparatus is not limited by what has been illustrated and described in the present specifications. It is intended that the specification and examples be considered as exemplary only, with a true scope of the apparatus being indicated by the following claims.

## Claims

1. An apparatus for skin treatment (100, 1100) said apparatus comprising:
a frame (104) configured to be applied to a treated skin segment (128, 300, 520, 704, 1004), said frame (104) including
at least one guide (108); and
a treatment head (116, 1000) configured to reciprocate on the at least one guide (108), the treatment head (116, 1000) including at least one light source (120, 604) configured to deliver light energy to a treated skin segment (128, 300, 520, 704, 1004); **characterised in that** the treatment head further comprises at least a pair of RF electrodes (504; 508, 800-1, 800-2, 800-3, 800-4); and
wherein in course of reciprocating movement (200, 400) of the treatment head (116, 1000) the light source (120, 604) operates to deliver through a light guide (124) light energy to the treated skin segment (128, 300, 520, 704, 1004) and when the pair of RF electrodes (504; 508, 800-1, 800-2, 800-3, 800-4) is fed with a low voltage, the pair of RF electrodes (504; 508, 800-1, 800-2, 800-3, 800-4) communicating with an electronic circuit (512) is configured to extract the treated skin segment (128; 300; 520; 704; 1004) impedance and when the pair of RF electrodes (504; 508, 800-1, 800-2, 800-3, 800-4) is fed with higher magnitude of RF energy as the low voltage, the pair of RF electrodes (504; 508, 800-1, 800-2, 800-3, 800-4) is configured to deliver RF energy to the treated skin segment (128; 300; 520; 704; 1004).

2. The apparatus (100, 1100) according to claim 1 wherein the treatment head (116, 1000) includes a mechanism configured to provide the treatment head position along the at least one guide (108) and wherein the mechanism is an encoder (132, 204).

3. The apparatus (100, 1100) according to claim 1 wherein the treatment head (116, 1000) includes a mechanism configured to provide a contact between the treatment head (116, 1000) and the treated skin segment (128, 300, 520, 704, 1004).

4. The apparatus (100, 1100) according to anyone of claims 1 and 3 wherein the treatment head is configured so that the reciprocating movement (200, 400) can be performed manually or with the help of a mechanism configured to provide to the treatment head reciprocating movement (200, 400).

5. The apparatus (100, 1100) according to claim 4 wherein the mechanism configured to provide to the treatment head reciprocating movement (200, 400) is an electric motor located in at least one handle (112) of frame (104) and a belt (208) conveying the movement to the treatment head (116).

6. The apparatus (100, 1100) according to anyone of claims 1 - 5 further comprising at least one sensor (500) configured to measure the treated skin segment temperature and wherein the sensor (500) is at least one of a group of sensors consisting of an infrared temperature sensor, a thermistor, a thermocouple, an electronic circuit configured to determine the treated skin segment impedance, and ultrasound sensor.

7. The apparatus (100, 1100) according to any one of claims 1 - 6 further comprising an optical sensor (600) configured to image the treated skin segment (128, 300, 520, 704, 1004) and assess the treated skin segment properties and wherein the skin properties include at least one of the skin colors.

8. The apparatus (100, 1100) according to any one of claims 1 - 7 further comprising a skin cooling device (212) associated with the treatment head (116, 1000) and configured to cool the treated skin segment (128, 300, 520, 704, 1004).

9. The apparatus (100, 1100) according to claim 8 wherein the skin cooling device (212) is located in one of a group of locations, consisting of a leading edge, middle of the treatment head and a trailing edge of the treatment head and wherein to facilitate skin treatment by reciprocating movement of treatment head (116; 1000) the leading edge and trailing edge of the treatment head (116; 1000) are made identical.

10. The apparatus (100, 1100) according to claim 1, wherein the frame (104) is made sufficiently rigid or equipped with vacuum cups facilitating stable location of frame (104) on skin in course of treatment process.

11. The apparatus (100, 1100) according to any one of claims 6 or 7 further comprising a controller (700) configured to communicate with at least one of the sensors (500, 600) and based on information received from the sensors (550, 600) change the treatment head movement speed, change the light energy, operate the skin cooling device (212), store at least one treatment protocol and provide to the light source (120, 604) and the RF electrodes (504, 508, 800), energy in accordance with the at least one treatment protocol.

12. The apparatus (100, 1100) according to any one of claims 1 - 11, wherein the at least a pair of RF electrodes (504, 508, 800-1, 800-2, 800-3, 800-4) are configured to be applied to surface to the treated skin segment (128, 300, 520, 704, 1004), maintain contact with the treated skin segment (128, 300, 520, 704, 1004) in course of the treatment head reciprocating movement (200, 400) and couple to the treated skin segment RF energy.

13. The apparatus (100, 1100) according to claim 8, wherein the pair of RF electrodes (504, 508, 800-1, 800-2, 800-3, 800-4) is located in one of a group of locations consisting of a location on both sides of a cooling device (212), on both sides of a light guide (124), and a combination of the above.

14. The apparatus (100, 1100) according to anyone of claims 1 - 13, wherein the controller (700) is also configured to control the RF energy coupled to the treated skin segment (128, 300, 520, 704, 1004).

15. The apparatus (100, 1100) according to anyone of claims 1 - 14 , wherein the controller (700) is also configured to store the at least one treatment protocol and provide to the light source (120, 604), the RF electrodes (504, 508, 800-1, 800-2, 800-3, 800-4), and the ultrasound transducers energy in accordance with the at least one treatment protocol.

## Patentansprüche

1. Eine Vorrichtung zur Hautbehandlung (100, 1100), wobei die Vorrichtung folgendes umfasst:
einen Rahmen (104), der konfiguriert ist, um an einen Abschnitt behandelter Haut (128, 300, 520, 704, 1004) angewendet zu werden, wobei der Rahmen (104) folgendes umfasst
mindestens eine Führung (108); und
einen Behandlungskopf (116, 1000), der konfiguriert ist, um sich an die mindestens eine Führung (108) hin und her zu bewegen, wobei der Behandlungskopf (116, 1000) mindestens eine Lichtquelle (120, 604) umfasst, die konfiguriert ist, um Lichtenergie an einen Abschnitt behandelter Haut (128, 300, 520, 704, 1004) zu liefern; **dadurch gekennzeichnet, dass** der Behandlungskopf folgendes umfasst
mindestens ein Paar von RF-Elektroden (504; 508, 800-1, 800-2, 800-3, 800-4); und
wobei bei der alternierenden Bewegung (200, 400) des Behandlungskopfes (116, 1000) die Lichtquelle (120, 604) einwirkt, um durch einen Lichtleiter (124) Lichtenergie an den Abschnitt behandelter Haut (128, 300, 520, 704, 1004) zu liefern und wenn das Paar von RF-Elektroden (504; 508, 800-1, 800-2, 800-3, 800-4) mit einer Niederspannung versorgt wird, das Paar von RF-Elektroden (504; 508, 800-1, 800-2, 800-3, 800-4), das mit einer elektronischen Schaltung (512) verbunden ist, konfiguriert ist, um Impedanz des Abschnitts behandelter Haut (128; 300; 520; 704; 1004) zu extrahieren und wenn das Paar von RF-Elektroden (504; 508, 800-1, 800-2, 800-3, 800-4) mit einem höheren Ausmaß an RF-Energie als die Niederspannung versorgt wird, das Paar von RF-Elektroden (504; 508, 800-1, 800-2, 800-3, 800-4) konfiguriert ist, um RF-Energie an den Abschnitt behandelter Haut (128; 300; 520; 704; 1004) zu liefern.

2. Die Vorrichtung (100, 1100) nach Anspruch 1, wobei der Behandlungskopf (116, 1000) einen Mechanismus umfasst, der konfiguriert ist, um die Position des Behandlungskopfes entlang der mindestens einen Führung (108) bereitzustellen und wobei der Mechanismus ein Encoder (132, 204) ist.

3. Die Vorrichtung (100, 1100) nach Anspruch 1, wobei der Behandlungskopf (116, 1000) einen Mechanismus umfasst, der konfiguriert ist, um einen Kontakt zwischen dem Behandlungskopf (116, 1000) und dem Abschnitt behandelter Haut (128, 300, 520, 704, 1004) bereitzustellen.

4. Die Vorrichtung (100, 1100) nach einem der Ansprüche 1 und 3, wobei der Behandlungskopf konfiguriert ist, so dass die alternierende Bewegung (200, 400) manuell oder mit Hilfe von einem Mechanismus durchgeführt werden kann, der konfiguriert ist, um dem Behandlungskopf die alternierende Bewegung (200, 400) zu verleihen.

5. Die Vorrichtung (100, 1100) nach Anspruch 4, wobei der Mechanismus, der konfiguriert ist, um dem Behandlungskopf die alternierende Bewegung (200, 400) zu verleihen ein Elektromotor ist, der in mindestens einem Griff (112) des Rahmens (104) und eines Bands (208), welches die Bewegung an den Behandlungskopf (116) überträgt, angeordnet ist.

6. Die Vorrichtung (100, 1100) nach einem der Ansprüche 1 - 5, weiterhin umfassend mindestens einen Sensor (500), der konfiguriert ist, um die Temperatur des Abschnitts behandelter Haut zu messen und wobei der Sensor (500) mindestens einer aus einer Gruppe von Sensoren ist, welche aus den folgenden besteht: einem Infrarottemperatursensor, einem Thermistor, einem Thermoelement, einer elektronischen Schaltung, welche konfiguriert ist, um die Impedanz des Abschnitts behandelter Haut festzustellen, und einem Ultraschallsensor.

7. Die Vorrichtung (100, 1100) nach einem der Ansprüche 1 - 6, weiterhin umfassend einen optischen Sensor (600), der konfiguriert ist, um den Abschnitt behandelter Haut (128, 300, 520, 704, 1004) abzubilden und die Eigenschaften des Abschnitts behandelter Haut zu prüfen und wobei die Hauteigenschaften mindestens eine der Hautfarben umfasst.

8. Die Vorrichtung (100, 1100) nach einem der Ansprüche 1 - 7 weiterhin umfassend eine Vorrichtung (212) zur Hautabkühlung, die mit dem Behandlungskopf (116, 1000) verknüpft ist und zur Abkühlung des Abschnitts behandelter Haut (128, 300, 520, 704, 1004) konfiguriert ist.

9. Die Vorrichtung (100, 1100) nach Anspruch 8, wobei die Vorrichtung zur Hautabkühlung (212) in einer aus einer Gruppe von Stellen angeordnet ist, welche aus einer Vorderkante, dem Mittelbereich des Behandlungskopfes und einer Hinterkante des Behandlungskopfes besteht und wobei die Vorderkante und die Hinterkante des Behandlungskopfes (116; 1000) identisch ausgestaltet sind, um die Behandlung der Haut mittels der alternierenden Bewegung des Behandlungskopfes (116; 1000) zu ermöglichen.

10. Die Vorrichtung (100, 1100) nach Anspruch 1, wobei der Rahmen (104) starr genug ausgestaltet ist oder mit Vakuumsaugern versehen ist, welche eine stabile Position des Rahmens (104) auf der Haut während des Behandlungsverfahrens ermöglichen.

11. Die Vorrichtung (100, 1100) nach einem der Ansprüche 6 oder 7, weiterhin umfassend eine Steuereinheit (700), die konfiguriert ist, und mit mindestens einen der Sensoren (500, 600) verbunden zu sein und die, ausgehend von aus den Sensoren (550, 600) empfangener Information die Geschwindigkeit des Behandlungskopfs zu variieren, die Lichtenergie zu variieren, die Vorrichtung (212) zur Hautabkühlung zu betätigen, mindestens ein Behandlungsprotokoll zu speichern und die Lichtquelle (120, 604) und die RF-Elektroden (504, 508, 800) gemäß dem mindestens einen Behandlungsprotokoll mit Energie zu versorgen.

12. Die Vorrichtung (100, 1100) nach einem der Ansprüche 1 - 11, wobei das mindestens ein Paar von RF-Elektroden (504, 508, 800-1, 800-2, 800-3, 800-4) konfiguriert ist, um an die Oberfläche des Abschnitts behandelter Haut (128, 300, 520, 704, 1004) angewendet zu werden, Kontakt mit dem Abschnitt behandelter Haut (128, 300, 520, 704, 1004) während der alternierenden Bewegung (200, 400) des Behandlungskopfes aufrechtzuerhalten und RF-Energie an den Abschnitt behandelter Haut anzukoppeln.

13. Die Vorrichtung (100, 1100) nach Anspruch 8, wobei das Paar von RF-Elektroden (504, 508, 800-1, 800-2, 800-3, 800-4) in einer Stelle aus einer Gruppe von Stellen angeordnet ist, welche aus den folgenden besteht: einer Position an beiden Seiten einer Abkühlvorrichtung (212), an beiden Seiten eines Lichtleiters (124), und einer Kombination von diesen.

14. Die Vorrichtung (100, 1100) nach einem der Ansprüche 1 - 13, wobei die Steuereinheit (700) auch konfiguriert ist, um die an den Abschnitt (128, 300, 520, 704, 1004) behandelter Haut angekoppelte RF-Energie zu steuern.

15. Die Vorrichtung (100, 1100) nach einem der Ansprüche 1 - 14, wobei die Steuereinheit (700) auch konfiguriert ist, um das mindestens eine Behandlungsprotokoll zu steuern und die Lichtquelle (120, 604), die RF-Elektroden (504, 508, 800-1, 800-2, 800-3, 800-4), und die Ultraschallwandler mit Energie gemäß des mindestens einen Behandlungsprotokolls zu versorgen.

## Revendications

1. Un appareil de traitement de la peau (100, 1100), comprenant ledit appareil :
un châssis (104) configuré pour être appliqué à un segment de peau traitée (128, 300, 520, 704, 1004), incluant ledit châssis (104)
au moins un guide (108) ; et
une tête de traitement (116, 1000) configurée pour produire un mouvement alternatif sur l'au moins un guide (108), la tête de traitement (116, 1000) incluant au moins une source de lumière (120, 604) configurée pour transmettre de l'énergie lumineuse à un segment de peau traitée (128, 300, 520, 704, 1004) ; **caractérisé en ce que** la tête de traitement comprend en outre
au moins une paire d'électrodes RF (504 ; 508, 800-1, 800-2, 800-3, 800-4) ; et
dans lequel pendant le mouvement alternatif (200, 400) de la tête de traitement (116, 1000) la source de lumière (120, 604) fonctionne pour délivrer au segment de peau traitée (128, 300, 520, 704, 1004) de l'énergie lumineuse à travers un guide de lumière (124) et dans lequel la paire d'électrodes RF (504 ; 508, 800-1, 800-2, 800-3, 800-4) est alimentée avec une basse tension, la paire d'électrodes RF (504 ; 508, 800-1, 800-2, 800-3, 800-4) communicant avec un circuit électronique (512) est configurée pour extraire de l'impédance du segment de peau traitée (128 ; 300 ; 520 ; 704 ; 1004) et lorsque la paire d'électrodes RF (504 ; 508, 800-1, 800-2, 800-3, 800-4) est alimentée avec une plus forte amplitude d'énergie RF que la basse tension, alors la paire d'électrodes RF (504 ; 508, 800-1, 800-2, 800-3, 800-4) est configurée pour délivrer de l'énergie RF au segment de peau traité (128 ; 300 ; 520 ; 704 ; 1004).

2. L'appareil (100, 1100) selon la revendication 1, dans lequel la tête de traitement (116, 1000) inclut un mécanisme configuré pour fournir la position de la tête de traitement le long de l'au moins un guide (108) et dans lequel le mécanisme est un encodeur (132, 204).

3. L'appareil (100, 1100) selon la revendication 1, dans lequel la tête de traitement (116, 1000) inclut un mécanisme configuré pour fournir un contact entre la tête de traitement (116, 1000) et le segment de peau traitée (128, 300, 520, 704, 1004).

4. L'appareil (100, 1100) selon l'une quelconque des revendications 1 et 3 dans lequel la tête de traitement est configurée de façon que le mouvement alternatif (200, 400) peut être effectué manuellement ou moyennant un mécanisme configuré pour fournir le mouvement alternatif (200, 400) à la tête de traitement.

5. L'appareil (100, 1100) selon la revendication 4, dans lequel le mécanisme configuré pour fournir le mouvement alternatif (200, 400) à la tête de traitement est un moteur électrique situé dans au moins une poignée (112) du châssis (104) et une bande (208) transmettant le mouvement à la tête de traitement (116).

6. L'appareil (100, 1100) selon l'une quelconque des revendications 1 - 5, comprenant en outre au moins un capteur (500) configuré pour mesurer la température du segment de peau traitée et dans lequel le capteur (500) est au moins un parmi groupe de capteurs constitué d'un capteur de température à infrarouge, une thermistance, un thermocouple, un circuit électronique configuré pour déterminer l'impédance du segment de peau traitée, et un capteur à ultrasons.

7. L'appareil (100, 1100) selon l'une quelconque des revendications 1 - 6, comprenant en outre un capteur optique (600) configuré pour imager le segment de peau traitée (128, 300, 520, 704, 1004) et évaluer les propriétés du segment de peau traitée et dans lequel les propriétés de la peau incluent au moins une des couleurs de la peau.

8. L'appareil (100, 1100) selon l'une quelconque des revendications 1 - 7, comprenant en outre un dispositif de refroidissement de la peau (212) associé avec la tête de traitement (116, 1000) et configuré pour refroidir le segment de la peau traitée (128, 300, 520, 704, 1004).

9. L'appareil (100, 1100) selon la revendication 8, dans lequel le dispositif de refroidissement de la peau (212) est situé dans un parmi un groupe d'emplacements constitué d'un bord avant, la région centrale de la tête de traitement et un bord arrière de la tête de traitement et dans lequel le bord avant et le bord arrière de la tête de traitement (116 ; 1000) sont réalisés identiques afin de faciliter le traitement de la peau moyennant le mouvement alternatif de la tête de traitement (116 ; 1000).

10. L'appareil (100, 1100) selon la revendication 1, dans lequel le châssis (104) est réalisé suffisamment rigide ou fourni avec des ventouses facilitant une position stable du châssis (104) sur la peau pendant le procédé de traitement.

11. L'appareil (100, 1100) selon l'une quelconque des revendications 6 ou 7 comprenant en outre un système de réglage (700) configuré de façon à communiquer avec au moins un des capteurs (500, 600) et à partir d'information provenant des capteurs (550, 600) modifier la vitesse du mouvement de la tête de traitement, modifier l'énergie lumineuse, faire fonctionner le dispositif de refroidissement de la peau (212), stocker au moins un protocole de traitement et fournir à la source de lumière (120, 604) et aux électrodes RF (504, 508, 800) de l'énergie selon l'au moins un protocole de traitement.

12. L'appareil (100, 1100) selon l'une quelconque des revendications 1 - 11, dans lequel l'au moins une paire d'électrodes RF (504, 508, 800-1, 800-2, 800-3, 800-4) est configurée pour être appliquée à la surface du segment de la peau traitée (128, 300, 520, 704, 1004), maintenir le contact avec le segment de la peau traitée (128, 300, 520, 704, 1004) pendant le mouvement alternatif (200, 400) de la tête de traitement et coupler de l'énergie RF au segment de la peau traitée.

13. L'appareil (100, 1100) selon la revendication 8, dans lequel la paire d'électrodes RF (504, 508, 800-1, 800-2, 800-3, 800-4) est située dans l'un parmi un groupe d'emplacements constitué d'un emplacement sur tous deux côtés d'un dispositif de refroidissement (212), sur tous deux côtés d'un guide de lumière (124), et une combinaison de ceux-ci.

14. L'appareil (100, 1100) selon l'une quelconque des revendications 1 - 13, dans lequel le système de réglage (700) est aussi configuré pour contrôler l'énergie RF couplée au segment de peau traitée (128, 300, 520, 704, 1004).

15. L'appareil (100, 1100) selon l'une quelconque des revendications 1 - 14, dans lequel le système de réglage (700) est aussi configuré pour stocker l'au moins un protocole de traitement et fournir à la source de lumière (120, 604), aux électrodes RF (504, 508, 800-1, 800-2, 800-3, 800-4), et aux transducteurs à ultrasons de l'énergie selon l'au moins un protocole de traitement.
